# EUROPEAN PATENT APPLICATION

(11) **EP 4 686 475 A1**
(43) Date of publication of application: **04.02.2026**
(21) Application number: 25192933.7
(22) Date of filing: 30.07.2025
(51) Int. Cl.: A61K 31/40, A61K 31/417, A61K 31/4418, A61K 31/444, A61K 31/609, A61K 33/243, A61K 45/06, A61P 35/00, A61K 31/282, A61K 31/555

(54) **XPG INHIBITORS FOR USE IN THE TREATMENT OF CANCER**

(30) Priority: 31.07.2024 PT 2024119633
(71) Applicant: FUNDAÇÃO D. ANNA DE SOMMER CHAMPALIMAUD E DR. CARLOS MONTEZ CHAMPALIMAUD - CENTRO DE INVESTIGAÇÃO DA FUNDAÇÃO CHAMPALIMAUD, 1400-038 Lisboa (PT); ASSOCIAÇÃO DA FACULDADE DE FARMÁCIA PARA A INVESTIGAÇÃO E DESENVOLVIMENTO, 1649-003 Lisboa (PT)
(72) Inventor: DE CASTILHO MONTEIRO GIL, NUNO JOSÉ, 1350-127 LISBOA (PT); DA ROCHA GUERREIRO OLIVEIRA, NUNO FILIPE, 2670-572 LOURES (PT); ÁLVARO MANGUINHAS, RITA CATARINA, 2910-433 SETÚBAL (PT); DO NASCIMENTO CARDOSO GUEDES, RITA ALEXANDRA, 1750-447 LISBOA (PT); ROSELL, RAFAEL, 08034 BARCELONA (ES); ALVES SERRA, PATRÍCIA FILIPA, 2695-622 SÃO JOÃO DA TALHA (PT)
(74) Representative: Monteiro Alves, Inês

(57) **Abstract**

The present invention discloses XPG inhibitor compounds and the use thereof in the treatment of cancer, in combination therapy with a platinum-based anticancer agent. The invention further extends to pharmaceutical compositions that comprise an effective amount of these compounds, as well as kits comprising the compounds and at least one platinum-based anticancer agent, therefore providing a tailored approach for the treatment of specific cancers, such as non-small cell lung cancer.

## Description

The present application claims the benefit of priority to the Portuguese provisional patent application no. 119633, filed on July 31, 2024, the contents of which is incorporated herein by reference.

### Technical Field

The present invention resides in the field of small-molecule organic compounds and encompasses a series of compounds which are identified for their therapeutic application as medicaments.

More specifically, the present invention discloses XPG inhibitors and the use thereof in the treatment of cancer, in combination with at least one anticancer agent, preferably a platinum-based anticancer agent.

The scope of the invention also extends to pharmaceutical compositions that comprise an effective amount of these compounds, as well as kits consisting of the compounds and at least one anticancer agent, therefore providing a tailored approach for the treatment of specific cancers, such as non-small cell lung cancer.

### Background Art

Non-small cell lung cancer (NSCLC), the predominant subtype of lung cancer (LC), remains a significant global health challenge, contributing to a substantial proportion of cancer-related deaths. Despite significant advances in treatment strategies, many NSCLC patients continue to face reduced survival rates due to factors such as late diagnosis, metastatic progression, and both intrinsic and acquired resistance to therapies.

Cells undergo genomic instability at multiple stages of their life cycle. As a result of this instability, DNA lesions, either from endogenous or exogenous sources, are constantly formed. To preserve genomic integrity, cells have evolved a series of DNA repair pathways, which are triggered to maintain genetic stability and integrity when cells are exposed to various types of DNA-damaging agents.

These complementary repair pathways are also crucial for cells to manage the endogenous DNA lesions that are continuously formed. Deregulation of these pathways can lead to the initiation and progression of cancer. Overexpression of several key players in DNA repair mechanisms has been identified as biomarkers of cancer progression and is associated with poorer prognosis.

Alterations in these pathways can contribute to either hypersensitivity or resistance of cancer cells to genotoxic agents. Since most current therapies depend on inducing cell death through direct or indirect DNA damage (e.g., ionizing radiation and chemotherapeutic agents), targeting specific components of DNA repair pathways can increase tumour sensitivity of these standard cancer treatments. This approach has led to an emerging field in cancer research that focuses on using pharmacological inhibitors of DNA repair enzymes / pathways to boost chemo / radiotherapy or as monotherapy.

Among the several pathways involved in DNA repair, the nucleotide excision repair (NER) pathway is responsible for removing bulky, helix-distorting DNA lesions such as those caused by UV light, environmental mutagens (e.g., benzo[a]pyrene or aromatic amines), and chemotherapeutic agents (e.g., platinum-based drugs, mitomycin C, carmustine, and nitrogen mustard). The NER pathway repairs among others, pyrimidine dimers, bulky DNA adducts, DNA-DNA crosslinks, and replication lesions.

Deficiencies in NER components result in xeroderma pigmentosum (XP), a skin cancer-prone inherited disorder characterized by increased UV sensitivity and mutagenicity in cells. Hence, the NER pathway holds significant clinical relevance in therapeutic contexts where DNA repair capacity may determine the treatment outcomes.

Within the research field of "DNA repair targeted therapy", several promising drugs have emerged, particularly targeting PARP1, and to a lesser extent O6-methyltransferase, ATM/DNA-PK, and APE1. However, there is a clear need for the development of improved NER inhibitors. Importantly, Computer-Aided Drug Design (CADD) approaches have also been utilized, primarily targeting ERCC1-XPF, XPA, and RPA, resulting in the identification of some direct inhibitors.

As critical components of the NER pathway, the structure-specific endonuclease activities of the ERCC1-XPF complex and the XPG protein are essential for repairing various DNA lesions, including intrastrand and interstrand crosslinks (ICLs). The ERCC1-XPF complex functions as a heterodimer, where XPF has the nuclease function of incising the DNA 5' to the lesion.

ERCC1, though catalytically inactive, regulates DNA-protein and protein-protein interactions. Given its involvement in repairing DNA damage induced by chemotherapeutics, the ERCC1-XPF complex is a promising druggable target to enhance chemotherapy efficacy and overcome resistance.

Overexpression of ERCC1-XPF has been linked with poorer prognosis and reduced response to chemotherapeutics in various cancers, including ovarian, testicular, non-small cell lung cancer, and squamous cell carcinoma. Altogether, the relevance of this target justifies the interest of the scientific community.

Despite numerous attempts to develop novel ERCC1-XPF inhibitors, many candidates have exhibited high off-target cytotoxicity, poor pharmacokinetic and physicochemical profile, and limited potency.

The importance of the NER pathway extends beyond ERCC1-XPF, as other targets within this pathway are equally critical. In this context, XPG is the endonuclease responsible for making the 3' incision at sites of DNA damage, being therefore a crucial component of the NER pathway. Knockdown studies of XPF and XPG (encoded by the ERCC5 gene) have shown a fivefold decrease in the repair efficiency of cisplatin / oxaliplatin-induced DNA damage, suggesting a potential for combinatory therapy.

This rationale relies on the fact that these endonucleases function sequentially and are both indispensable for the completion of NER. Moreover, ERCC5 downregulation has been identified as a novel prognostic biomarker for platinum-treated ovarian cancer patients. However, so far, no study has been carried out with the purpose of identifying XPG inhibitors.

Lung cancer (LC) is the leading cause of cancer-related deaths for both men and women, with NSCLC accounting for approximately 85% of all LC cases. Unlike the steady increase in survival rates observed for most cancers, progress has been limited for LC, with the current 5-year relative survival rate hovering around 20%. Platinum-based adjuvant therapies are typically the first-line treatment, yet resistance to these therapies is common.

Given the results observed in other types of cancers and the absence of existing inhibitors, the present invention aims to identify XPG inhibitors for use in the treatment of cancer, especially in combination therapy with platinum-based agents in NSCLC patients to improve clinical outcomes.

This involved characterizing the biological target's structure, compiling reported inhibitors, selecting an appropriate docking protocol, and conducting a virtual screening campaign. Post-screening, potential inhibitors were acquired from the ChemBridge database and tested in a NSCLC cell line with high ERCC5 expression. A pre-clinical safety assessment was also performed using a non-tumoral lung cell line to identify the safest inhibitors capable of enhancing cisplatin-induced cytotoxicity.

### Prior art documents

It is already disclosed in the prior art that the NER's role in protecting cells from DNA damage can contribute to cancer cell survival by repairing DNA damage caused by chemotherapy, leading to drug resistance.

The prior art documents collectively highlight the ongoing efforts to target the NER pathway as a potential cancer treatment strategy, either to enhance the effectiveness of chemotherapy or to directly target cancer cells with NER deficiencies.

Prior art document US20210093730A1 discloses biomarkers useful to predict efficacy and / or toxicity of antibody-drug conjugates (ADC) therapy, determine tumor response to treatment, identify minimal residual disease or relapse, determine prognosis, stratify patients for initial therapy or optimize treatment for the patient, based on the specific biomarkers detected.

Prior art document US20240207300A1 relates to methods for the treatment of cancer based on the administration of combination therapies including myt1 inhibitors and an ATR inhibitor.

Prior art document US2020237763A1 discloses compounds capable of modulating the level of activity of nucleases such as Flap structure-specific endonuclease 1 (FEN1), Xeroderma Pigmentosum Complementation Group G (XPG) protein, Exonuclease 1 (EXO1) and/or GEN1 and methods of using the same.

Prior art document US11584755B2 contemplates the use of the FEN1, EXO1 or XPG antagonists (and not inhibitors) in the treatment or prevention of a broad range of diseases, such as cancer.

In articles "DNA repair pathways and their therapeutic potential in lung cancer" (Burgess, JT (2014)) and "The therapeutic potential of DNA damage repair pathways and genomic stability in lung cancer" (Burgess, JT (2020)), the authors address the association between lung cancer disease - occurrence and progression - and genetic instability, with a central focus on genetic mutations in the DNA damage repair pathways.

In this context, a structure-based virtual screening approach was adopted, including a structural and physicochemical analysis of the XPG protein, and a library of small molecules with reported inhibitory activities towards XPG was retrieved and investigated.

Thus, it is an object of the present invention to find compounds which are useful as XPG inhibitors in a specific way, therefore reducing off-target effects and minimizing toxicity to normal cells.

### Summary of Invention

In a first aspect of the present invention, it is disclosed a XPG inhibitor compound and / or a pharmaceutically acceptable salt, solvate or tautomer thereof for use as a medicament in the treatment of cancer, tumors and / or metastases in combination with at least one anticancer agents, preferably a platinum-based anticancer agent.

In a second aspect of the present invention, it is disclosed a pharmaceutical composition comprising an effective amount of at least one XPG inhibitor compound and / or a pharmaceutically acceptable salt, solvate or tautomer thereof and excipients.

In a third aspect of the present invention, it is disclosed a kit consisting of separate packs of (a) an effective amount of a XPG inhibitor compound and / or a pharmaceutically acceptable salt, solvate or tautomer thereof, and (b) an effective amount of at least one anticancer agent.

### Technical Problem

The XPG protein (also known as ERCC5) plays a key role in DNA repair, particularly in the NER pathway. This pathway is responsible for removing bulky DNA lesions, such as those caused by UV light or chemotherapeutic agents like cisplatin.

However, XPG protein is not specific to normal cells or cancer cells - it is a general DNA repair protein that functions wherever it is expressed, repairing DNA damage in all cells that produce it, whether they are healthy, cancerous, or otherwise abnormal.

Proper XPG function protects against mutations and cancer development. However, in the therapeutic context, inhibiting XPG in cancer cells can be advantageous, as it sensitizes tumors to DNA-damaging agents, thus increasing the efficacy of chemotherapy.

Thus, there is a need in providing improved therapeutic strategies or agents for the treatment of cancer, particularly by enhancing the efficacy of platinum-based chemotherapy or overcoming resistance associated with such treatment.

### Solution to Problem

The present invention provides a solution to the above problem by identifying and providing novel and efficient XPG inhibitors compounds capable of sensitizing cancer cells to platinum-based agents, while maintaining acceptable selectivity and safety profiles.

The scope of the invention also extends to pharmaceutical compositions that comprise an effective amount of these compounds, as well as kits consisting of the compounds and at least one anticancer agent, providing a tailored approach for the treatment of specific cancers.

### Advantageous Effects of Invention

By inhibiting XPG-mediated DNA repair, the compounds of the present invention increase the sensitivity of cancer cells to DNA-damaging agents, making them more susceptible to DNA damage-induced apoptosis.

The compounds disclosed in the present invention reduce the cell's ability to repair platinum-induced DNA lesions and their applicability across different chemotherapy regimens is broadened, leading to improved treatment outcomes.

### Brief Description of Drawings

In order to facilitate an understanding of the principles according to the embodiments of this invention, reference will be made to the illustrated embodiments in the Figures and the language used to describe them.

It should also be understood that there is no intention to limit the scope of the invention to the content of the Figures and that modifications to the inventive features illustrated herein, as well as additional applications of the illustrated principles and embodiments, which would normally occur to a person skilled in the art having possession of this description, are considered within the scope of the claimed invention.
**Fig.1**

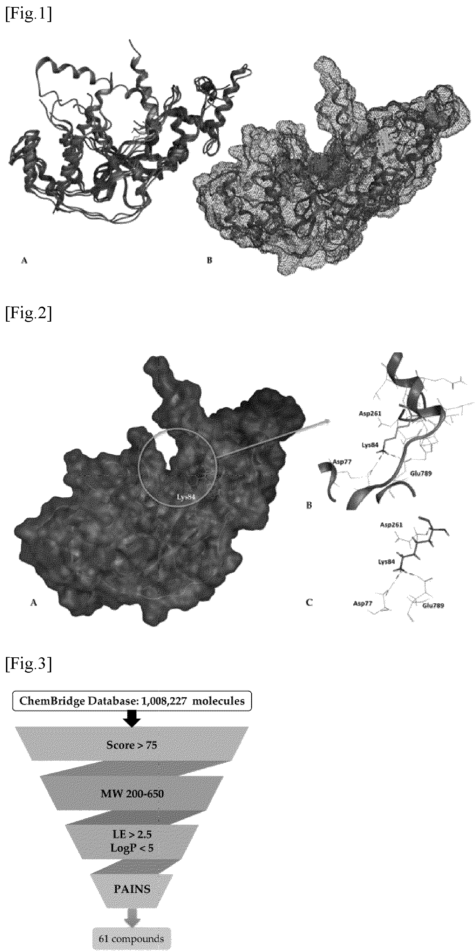

   [Fig. 1] depicts the XPG structure and surface representation, wherein (A) shows the overlap of XPG structures from PDB IDs: 6TUR, 6TUX, and 6VHB and (B) shows the molecular surface representation of XPG, highlighting the active site and alternative cavities.
**Fig.2**
   [Fig.2] depicts the XPG protein structure (6TUR), wherein (A) is a representation of the XPG 6TUR protein surface, highlighting the location of the binding pocket; (B) is the region of the XPG structure with emphasis on the pocket cavity and residue Lys84; and (C) is a close-up of Lys84 at the pocket entrance, showing its interaction with nearby residues.
**Fig.3**
   [Fig.3] is a schematic representation of the filtering criteria applied for compound selection from the ChemBridge database.
**Fig.4**

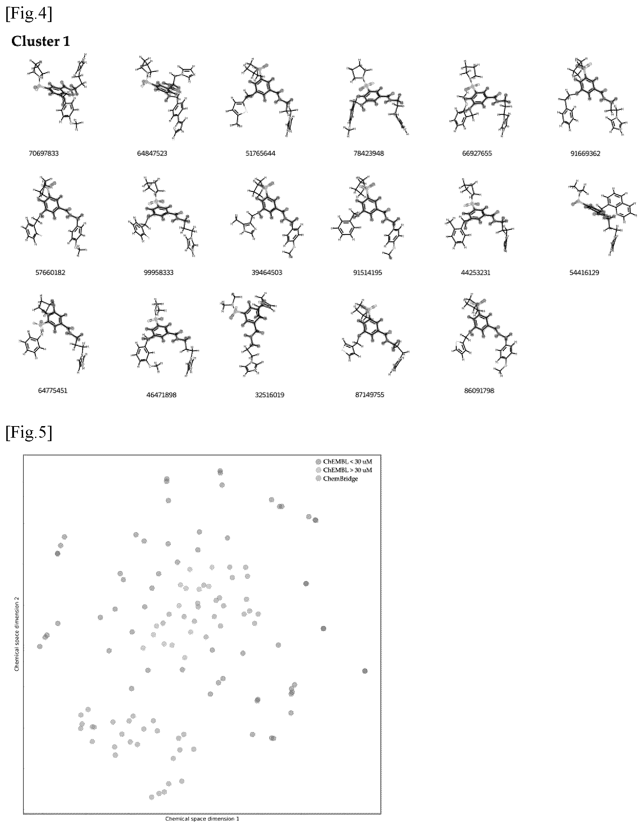

   [Fig.4] is a representation of cluster 1 derived from the ChemBridge results dataset proposed from the visual inspection, wherein the highlighted parts of the molecules represent the similar scaffold that defines each cluster.
**Fig.5**
   [Fig.5] depicts a t-SNE distribution representing the chemical diversity of active and inactive compounds from ChEMBL. Additionally, the hit molecules from the ChemBridge database are also represented.
**Fig.6**

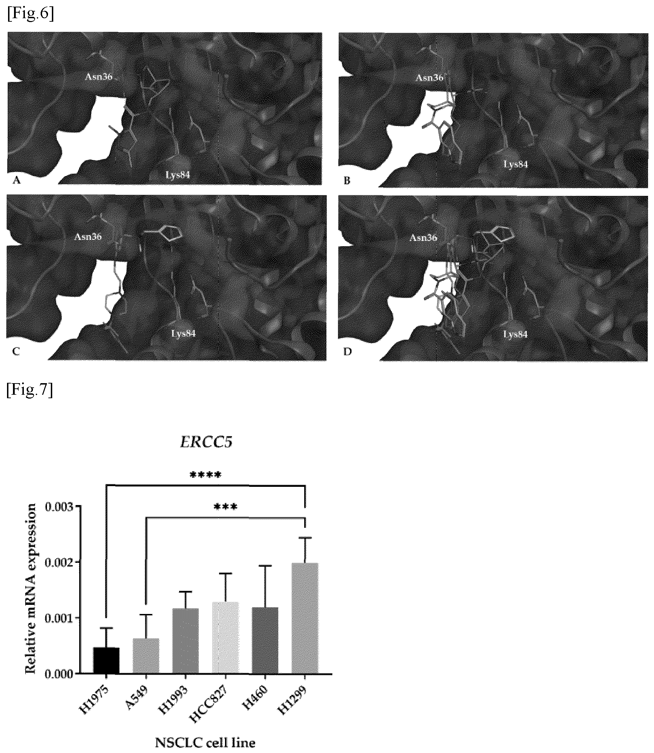

   [Fig.6] is a representation of compounds docked into the XPG protein binding pocket, wherein (A) is the XPG highlighting the Lys84 and Asn36 residues inside the binding pocket; docked complexes of XPG with potential inhibitor CB41, (B) are the docked complexes of XPG with potential inhibitor CB60, (C) are the docked complexes of XPG with potential inhibitor CB22, and (D) is the overlap of the three small molecules. All these molecules fit snugly within the pocket, effectively blocking access to the top of the cavity.
**Fig.7**
   [Fig.7] shows ERCC5 relative mRNA expression levels (β-actin) for all the representative NSCLC cell lines. Relative expression was calculated using the expression 2-ΔCt, where ΔCt = Average Ct (gene of interest) - Average Ct (β-ACTIN) and normalized it to the reference gene β-ACTIN. The results are expressed as mean ± SD (n=3). ***p < 0.001 and ****p < 0.0001 (one-way ANOVA, Tukey's multiple comparisons test).
**Fig.8**

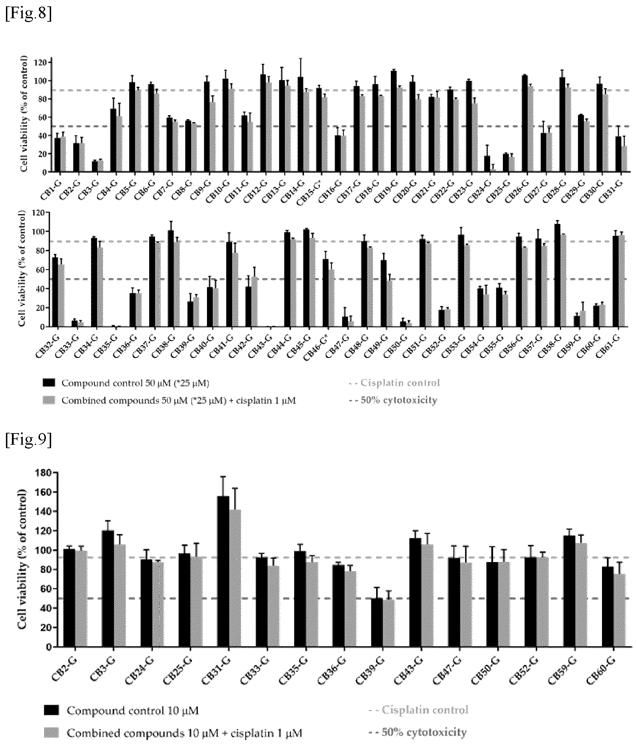

   [Fig.8] shows the sensitizing effect of the acquired XPG inhibitors on cisplatin-induced cytotoxicity. Compounds marked with * were tested at 25 µM due to solubility issues. Values represent mean ± SD and are expressed as percentages of the vehicle-treated control cells considered as 100% of cell viability (n=2-3).
**Fig.9**
   [Fig.9] shows the sensitizing effect of the acquired XPG inhibitors, which were considered extremely cytotoxic at 50 µM, on cisplatin-induced cytotoxicity. Values represent mean ± SD and are expressed as percentages of the vehicle-treated control cells which were considered as 100% of cell viability (n=2-3).
**Fig.10**

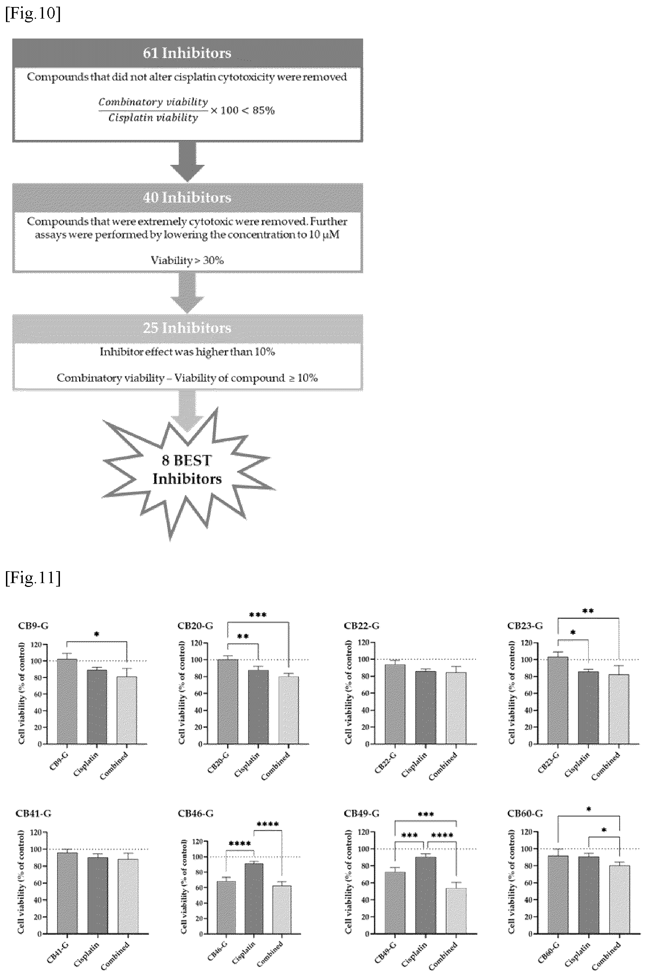

   [Fig.10] shows the strategy employed to select the 8 best inhibitors.
**Fig.11**
   [Fig.11] depicts the effect of the 8 best inhibitors in sensitizing H1299 cells to cisplatin-induced cytotoxicity. Values represent mean ± SD and are expressed as percentages of the vehicle-treated control cells considered as 100% of cell viability, identified as the dotted line in the graphs (n=3-5). *p < 0.05, **p < 0.01, ***p < 0.001, and ****p < 0.0001 (one-way ANOVA, Tukey's multiple comparisons test).
**Fig.12**

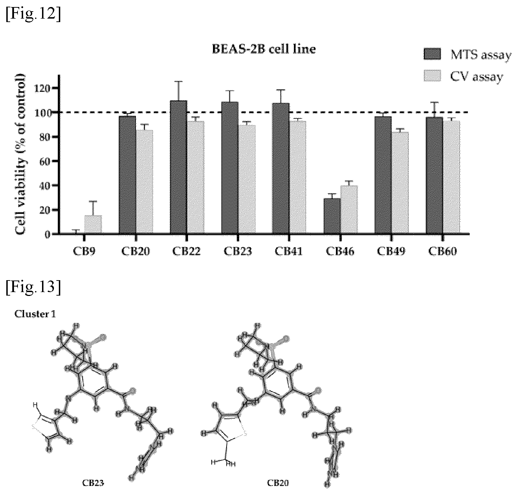

   [Fig.12] depicts the cytotoxicity of the 8 best inhibitors in BEAS-2B cells. Values represent mean ± SD and are expressed as percentages of the vehicle-treated control cells considered as 100% of cell viability, identified as the dotted line in the graph (n=2-3).
**Fig.13**
   [Fig.13] is a representation of cluster derived from the final data set of 8 molecules proposed as inhibitors. Between the 8 molecules, only two were similar to be considered as a cluster. Parts of the molecules highlighted in red represent the similar scaffold that defines each cluster.
**Fig.14**

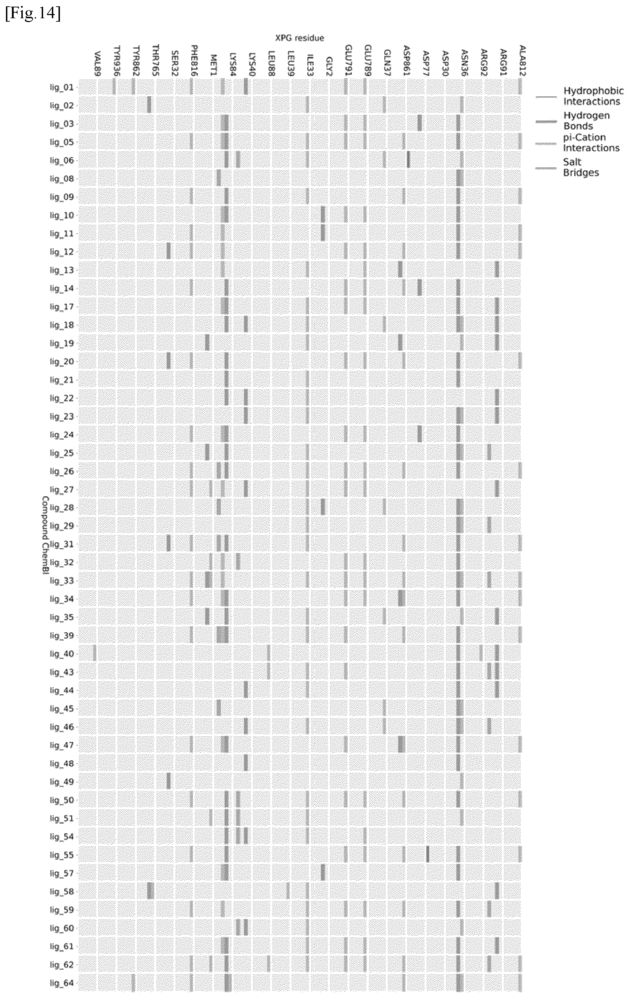

   [Fig.14] is a representation of the types of interactions established between the XPG protein and the compounds retrieved from ChemBL with activity from 23 nM up to 30 µM (50 compounds). Each column represents the XPG residues, while the rows identify each of the 50 molecules.
**Fig.15**

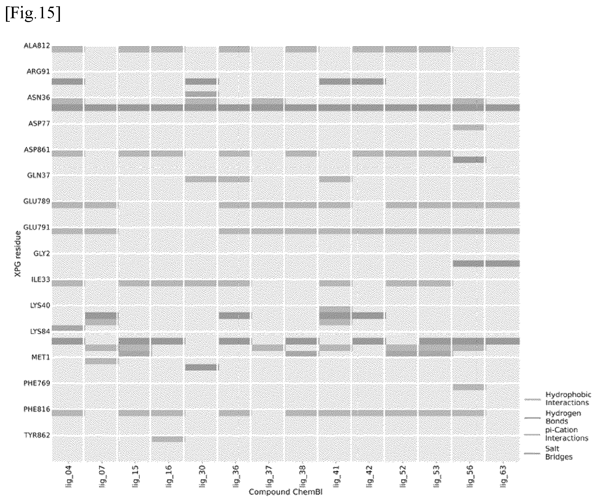

   [Fig.15] is a representation of the types of interactions established between the XPG protein and the compounds from ChemBL with activity superior to 30 µM (14 compounds). Each row represents the XPG residues, while the columns identify each of the 50 molecules.
**Fig.16**

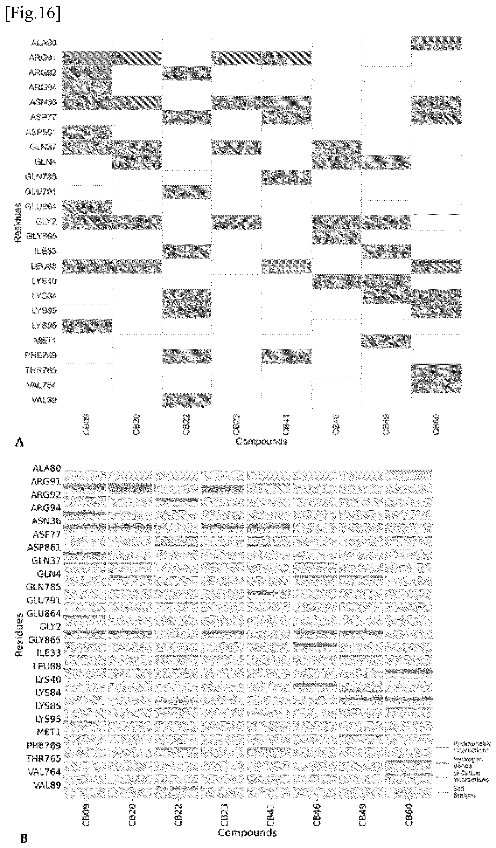

   [Fig.16] shows (A) XPG residues interacting with the top 8 compounds, wherein rows represent specific XPG residues, and columns represent active compounds from VS. Grey Squares indicate ligand-receptor interactions, while the white squares indicate no interactions; (B) interaction types between XPG protein and active compounds from VS. Rows represent XPG residues and columns represent the 8 active molecules.

### Description of Embodiments

The present invention discloses, in a first aspect, XPG inhibitors compounds that enhance the cytotoxicity induced by platinum-based compounds.

The molecules that are already known to display activity against XPG were primary derived from 2,4-diketobutyric acid and 3,4-diketobutyrates, with various substitutions.

Analysis of three-dimensional (3D) structures of the XPG protein and a comprehensive search of the ChEMBL28 database was conducted to retrieve reported small-molecules that could target XPG.

In the present invention, novel molecules for XPG inhibition were identified. The selection of a given compound to proceed in further biological tests was based on multiple criteria, including scoring metrics, ligand efficiency, binding pocket fit, and interactions with adjacent residues. Thus, the XPG inhibitors compounds of the present invention comprise:
at least one optionally substituted aromatic or heteroaromatic ring;
none or 1 hydrogen bond donors selected from at least one of a hydroxyl group, a ketone group, a thiol group and a nitrogen-containing group;
at least 4 hydrogen bond acceptors selected from at least one of oxygen, nitrogen and sulfur;
wherein the molecular weight ranges between 200 and 300 g/mol;
and / or a pharmaceutically acceptable salt, solvate or tautomer thereof for use as a medicament in the treatment of cancer, tumors and / or metastases in combination with at least one platinum-based anticancer agent, wherein the compound and the Lys84 residue of the XPG protein participate in a molecular interaction to inhibit XPG activity.

In a more preferred embodiment of the present invention, the XPG inhibitors compounds comprise:
at least one optionally substituted phenyl, furanyl, pyrrolyl, thienyl, thiophenyl, oxazolyl, indazolyl, isoxazolyl, thiazolyl, isothiazolyl, imidazolyl, pyrazolyl, triazolyl, pyridyl, pyridinyl, pyrazolyl, dihydrothiadiazolyl, dioxazolyl, oxadiazolyl, pyrazinyl, pyrimidinyl, pyridazinyl, piperazinyl, triazinyl, indolyl, quinolinyl, isoquinolinyl, purpyrrolyl, benzimidazolyl, benzoxazolyl, benzothiazolyl, pteridinyl, azepanyl, diazinanyl, pyrrolidinyl, imidazolyl, imidazolidinyl, morpholinyl or other mono- or poly aromatic or non-aromatic homocyclic or heterocyclic compound;
none or 1 hydrogen bond donors selected from at least one of a hydroxyl group, a ketone group, a thiol group and a nitrogen-containing group selected from substituted or unsubstituted amino group selected from primary or secondary amine or amide;
at least 4 hydrogen bond acceptors selected from at least one of oxygen, nitrogen and sulfur, the oxygen being found in carbonyl groups, hydroxyl groups, ether groups or esters groups, the nitrogen being found in amines, amides and heteroaryl groups, and the sulfur being found in sulfonyl and thiocarbonyl groups;
wherein the molecular weight ranges between 200 and 300 g/mol;
and / or a pharmaceutically acceptable salt, solvate or tautomer thereof for use as a medicament in the treatment of cancer, tumors and / or metastases in combination with at least one platinum-based anticancer agent, wherein the compound and the Lys84 residue of the XPG protein participate in a molecular interaction to inhibit XPG activity.

An initial screening approach was developed in vitro and comprised the identification of the XPG putative inhibitors that could in fact enhance cisplatin-induced cytotoxicity in H1299 cells which exhibited higher ERCC5 expression.

From all the tested compounds, 8 of them demonstrated positive results in inhibiting XPG and enhancing cisplatin-induced cytotoxicity. All these compounds are simple small molecules with good spatial fitting in the XPG active binding site and establish important interactions with Lys84 residue.

In a preferred embodiment of the present invention, the XPG inhibitor compounds are selected from:
2-(2-{[4-(acetylamino)phenyl]amino}-2-oxoethoxy)-N-[2-(2-isopropyl-5-methylphenoxy)ethyl]benzamide 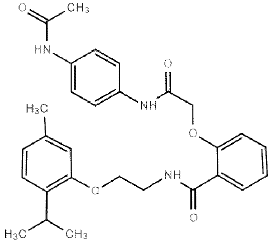
N-[3-(1H-imidazol-1-yl)propyl]-3-{[(5-methyl-2-thienyl)methyl]amino}-5-(1-pyrrolidinylsulfonyl)benzamide 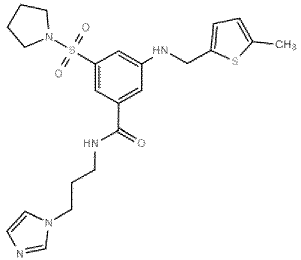
1-[3-(2-{4-[(3,5-dimethyl-1H-pyrazol-1-yl)acetyl]-1-piperazinyl}ethoxy)phenyl]-N-methyl-N-(2-thienylmethyl)methanamine 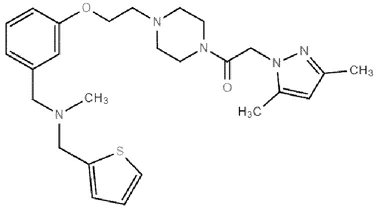
N-[3-(1H-imidazol-1-yl)propyl]-3-(1-pyrrolidinylsulfonyl)-5-[(3-thienylmethyl)amino]benzamide 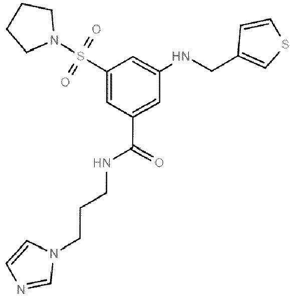
N-({8-[4-(3-hydroxy-3-methyl-1-butyn-1-yl)benzyl]-1-oxa-8-azaspiro[4.5]dec-2-yl }methyl)-1,3-dimethyl-1H-pyrazole-5-carboxamide 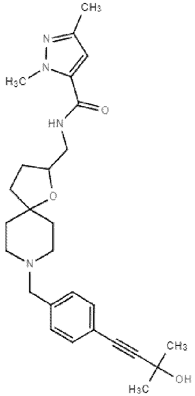
1,1'-[1,4-phenylenebis(oxy-4,1-phenylene)]di(2,5-pyrrolidinedione) 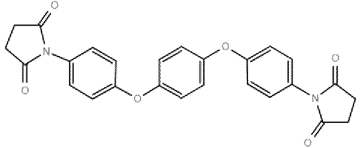
Methyl 1-benzyl-5-[(cyclohexylmethyl)amino]-3-[(3-pyridinylcarbonyl) amino]-1H-pyrrolo[2,3-b]pyridine-2-carboxylate 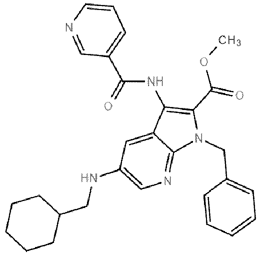
N-[(2R*,3R*)-2-(benzyloxy)-1'-(1H-indol-6-ylcarbonyl)-2,3-dihydrospiro[indene-1,4'-piperidin]-3-yl]acetamide 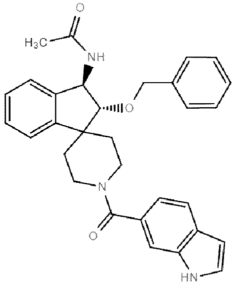
and / or a pharmaceutically acceptable salt, solvate or tautomer thereof for use as a medicament in the treatment of cancer, tumors and / or metastases in combination with at least one platinum-based anticancer agent, wherein the compound and the Lys84 residue of the XPG protein participate in a molecular interaction to inhibit XPG activity.

In preferred embodiments of the present invention, the platinum-based anticancer agent is selected from the group consisting of cisplatin, carboplatin, oxaliplatin, nedaplatin, lobaplatin, satraplatin, and heptaplatin.

In preferred embodiments of the present invention, the XPG inhibitor compounds and / or pharmaceutically acceptable salt, solvate or tautomer thereof is used in the treatment of non-small cell lung cancer.

In a second aspect of the present invention, it is disclosed a pharmaceutical composition comprising an effective amount of at least one XPG inhibitor compounds or a pharmaceutically acceptable salt, solvate or tautomer thereof and excipients. In preferred embodiments of the present invention, the pharmaceutical composition further comprises at least one platinum-based anticancer agent selected from the group consisting of cisplatin, carboplatin, oxaliplatin, nedaplatin, lobaplatin, satraplatin, and heptaplatin.

In a third aspect of the present invention, it is disclosed a kit comprising of separate packs of
(a) an effective amount of a XPG inhibitor compounds and / or a pharmaceutically acceptable salt, solvate or tautomer thereof, and
(b) an effective amount of at least one platinum-based anticancer agent selected from the group consisting of cisplatin, carboplatin, oxaliplatin, nedaplatin, lobaplatin, satraplatin, and heptaplatin.

Overall, the present invention highlights the importance of addressing the key features of small molecules to guide the discovery of XPG inhibitors, therefore providing novel compounds to be used in combination with platinum-based chemotherapy in cancer, more specifically, cisplatin therapy in non-small cell lung cancer.

### Examples

In the present invention, a structure-based virtual screening strategy targeting the inhibition of XPG was developed. Using the optimized docking protocol described below, the compounds were screened and then validated in H1299 cells, a NSCLC cell line exhibiting the highest ERCC5 expression.

The MTS assay was performed to determine whether inhibitors could enhance cisplatin-induced cytotoxicity, and 8 compounds proved to be promising candidates. Their inherent cytotoxicity was further investigated in a non-tumoral lung cell line (BEAS-2B cells).

Lys84 was identified as a critical residue for XPG activity and targeting this residue with small molecules demonstrated significant potential for inhibiting endonuclease function.

### Selection and preparation of XPG protein structures

Understanding structural properties, binding regions, flexibility, key interactions within binding pockets, and dynamics of ligand-receptor interactions are essential for drug development.

XPG plays a pivotal role in DNA damage repair via the NER pathway. Structurally, XPG is characterized by the N- and C-subdomains, and a helix-2turn-helix domain (H2TH) that forms the K+ binding site and β-pin, which is responsible for DNA interaction.

As an endonuclease, XPG belongs to a superfamily with a conserved catalytic domain comprising of two regions, "N" and "I". What sets XPG apart from other endonucleases is the insertion of 680 amino acids, known as the R-domain or spacer region, and an extended C terminus after the "I" region. These insertions interfere with the crystallization process due to predicted coiled coils and disordered regions.

The 3D structure of XPG features a core with a higher presence of β-sheets, a seven-stranded twisted β-sheet center, and α-helices connecting different regions, impacting both the DNA binding region and the active site. The active site is characterized by a high number of carboxylates, connecting the N- and I-subdomains and involving the spacer region, which functions as flexible linker to facilitate the native configuration of the structure.

Seven three-dimensional (3D) structures of the XPG protein were available in the Protein Data Bank (PDB) (Table 1 below) and support this observation, showing gaps and requiring careful selection for docking studies. None of these structures included small-molecule inhibitors, and two were complexes interacting with DNA.

All selected crystal structures represent the human variant of XPG, except two from Mus musculus (PDB IDs: 5EKG and 5EKF). Five structures featuring the XPG endonuclease catalytic domain were selected for simulations and analysis, specifically PDB IDs: 6VBH, 6TUR, 6TUS, 6TUX, and 6TUW. These structures were retrieved from PDB and prepared using the Molecular Operating Environment (MOE v.2020.0901, MOE from now on) structure preparation module. Hydrogen atoms were added, and appropriate protonation states were assigned using the Protonate-3D tool at pH 7.4 within MOE software. Crystallographic water molecules and other non-relevant molecules were removed.

**Table 1. List of X-ray structures of the XPG protein available in the PDB. The structure highlighted in bold was used for further analysis in this study.**

| **PDB ID** | **Res. (Å)** | **Year** | **Information** | **Reference** |
|---|---|---|---|---|
| 6VBH | 2.00 | 2020 | Human XPG endonuclease catalytic domain | NPL4 |
| **6TUR** | **2.90** | **2020** | **Human XPG, Apo1 form** | **NPL5** |
| 6TUS | 2.50 | 2020 | Human XPG, Apo2 form | NPL5 |
| 6TUX | 3.10 | 2020 | Human XPG-DNA, Complex 2 | NPL5 |
| 6TUW | 3.50 | 2020 | Human XPG-DNA, Complex 1 | NPL5 |
| 5EKF | 2.00 | 2016 | Mus musculus XPG complexed with Importin-alpha, fragment 1 | NPL3 |
| 5EKG | 2.80 | 2016 | Mus musculus XPG complexed with Importin-alpha, fragment 2 | NPL3 |

The non-human structures were discarded, and the remaining ones were categorized into apo structures and XPG-DNA complexes.

The structural characterization of XPG has provided insights into its role in DNA repair. High-resolution crystallography has elucidated the complex architecture of XPG, highlighting the importance of its endonuclease and DNA-binding domains, interdomain linkers, and protein-protein interaction regions. Understanding these structural features is crucial for deciphering the molecular mechanisms underlying XPG's function and developing therapeutic strategies targeting its function.

Like other endonucleases, XPG has the potential to bind to DNA via its active site, which was the primary focus of this study. The active site forms an access point between the helices, making it a critical region for targeting.

The docking binding site center was anchored using Lys84, Asn36, and Asp862 residues. These key residues were identified through visual inspection and CAVIAR (CAVity Identification and Rationalization), an open-source tool designed for generating descriptors for binding sites and detecting protein cavities using 3D structures. The docking region was defined with a 15 Å radius around these residues. All scoring functions were assessed for their efficacy in scoring and ranking, with ChemPLP ultimately chosen for subsequent virtual screening campaigns, utilizing 500 GA runs and centering on the Lys84 residue.

For the docking studies, the cavity around Lys84 was selected as the binding site due to its high conservation across the XPG superfamily and its ordered positioning (Figure 1A). Structural analysis indicated that the residue Lys84 is crucial for maintaining XPG activity since modifying this residue could inhibit the endonuclease function. Lys84 is strategically positioned in a central cavity formed by the protein, making it an ideal target for docking calculations, now referred to as the Lys84 cavity (red surface in Figure 1B).

The region defined by the Lys84 cavity is conserved across all available 3D structures of XPG, with the proximity zone of this residue being best preserved in the 6TUR structure. No previous computational studies targeting this protein have been reported, and no crystallographic structure of XPG complexed with an inhibitor is available. This gap underscores the novelty and potential impact of research in identifying inhibitors for XPG.

In the initial analysis, the retrieved 3D structures of XPG proteins (Table 1) were aligned and superimposed. Figure 1A shows the overlap of the 6TUR, 6TUX and 6VHB structures. The root mean square deviations (RMSDs) of the structures ranged from 0.67 Å to 1.93 Å, indicating high structural similarity. The overlap revealed significant resemblance, particularly around the putative binding pocket region defined around Lys84, as identified using MOE. The pocket surrounding Lys84 is highly conserved, suggesting its potential as a key site for inhibiting endonuclease activity. In the 6VHB structure, the RMSD spiked to 3.97 Å, and more gaps were observed.

When comparing the two apo structures, 6TUR and 6TUS, it was found that in 6TUR, Lys84 is positioned deeply within the pocket, which is advantageous for docking calculations (Figure 2). In this structure, Lys84 interacts with Asp77, Glu789, and Asp861, forming H-bond interactions. Conversely, in 6TUS, Lys84 is near a gap in the structure (with residue 92 absent), which could mask the 3D conformation, leading to its exclusion.

In structures 6TUX and 6TUW, where XPG is linked to a DNA fragment, structural rearrangements occur due to the binding. These changes justified the decision to proceed with the apo structure for further studies. Using an apo receptor is rationalized by the hypothesis that interference with the DNA repair mechanism can be better understood without DNA-induced conformational changes. The absence of residue 92 in 6TUS further supports the use of the 6TUR apo form for accurate analysis. Therefore, the apo structure 6TUR was selected to mimic the stage before DNA interaction, favouring inhibition and disruption of the repair mechanisms.

An independent analysis using CAVIAR confirmed the selected binding pocket around Lys84 as the highest-scoring cavity with a size of 280 Å3, a hydropathicity of 36%, and a drug binding site classification of 0.6/1 (Figure 1B). This classification supports the high potential of this cavity as a drug binding site. CAVIAR identified a total of six potential cavities in the XPG structure (cavities coloured yellow at figure 1B).

### Assembly of an XPG small molecule inhibitors set

A comprehensive search of the ChEMBL28 database was conducted to retrieve reported small-molecules that could target XPG and a group of 64 molecules was identified.

This collection was chosen to validate the docking protocol to be used in the virtual screening calculations. The molecules obtained from ChEMBL exhibited a wide range of inhibitory activities towards XPG and were reported in two previous studies specifically focused on the discovery of Flap endonuclease-1 (FEN1) inhibitors.

In those studies, the XPG activity was also evaluated to check for enzyme specificity. The molecules that were also identified to display activity against XPG were primary derived from 2,4-diketobutyric acid and 3,4-diketobutyrates, with various substitutions. These compounds were used to validate the molecular docking protocol for the virtual screening campaign described below.

An exhaustive analysis was conducted, focusing on various properties and descriptors, including molecular weight, H-bond acceptors and donors, logP, and Lipinski violations, among others. Molecular descriptors were computed using FAFDrugs4 and MOE, encompassing both conventional and pharmacophore-based descriptors aligned with drug-likeness rules, ideal drug attributes, and medicinal chemistry properties.

The structural diversity of these molecules was significant, with molecular weights ranging from less than 200 g/mol to 412 g/mol and a wide range of logP values, reflecting their different lipophilicity. The logP values in the dataset were up to 4, and all molecules adhered to the Lipinski rule of 5.

When examining the dataset of compounds with activities up to 30 µM, defined as active from the ChEMBL dataset, it was observed that the logP values predominantly ranged between 1 and 3 (1.5-2.5 more exactly), the molecular weights primarily fell between 200 to 300 g/mol, with 0 to 1 H-bond donors and at least 4 H-bond acceptors, without any violations of Lipinski's rule of 5.

This comprehensive analysis, which presents features indicating a good drug like profile focused on Lipinski's rule, is crucial for selecting compounds after virtual screening, offering a foundation for further biological assays and enhancing the understanding of the molecular characteristics that influence XPG inhibition.

### Selection of the docking protocol

The establishment and validation of an optimal docking protocol for subsequent virtual screening campaigns in an extensive molecular database began with a pivotal step, a series of docking simulations. After selecting the dataset of small molecules and the receptor structure, docking calculations were performed to validate the protocol for virtual screening.

The docking search space was defined to encompass the interaction region around the Lys84 residue. Lys84 was tested as the center of the search space, along with two additional residues that were also evaluated for their potential as binding pocket centers. Comprehensive analyses and comparisons were conducted on all results from these molecular docking calculations to identify and validate the most accurate point that best reproduces the available experimental data.

Additionally, four widely recognized scoring functions available in the GOLD v5.07 software suite (ChemPLP, GoldScore, ChemScore, and ASP) were assessed. In this validation process, the previously prepared ChEMBL dataset was docked into the selected XPG 3D structure within a 15 Å radius of the identified binding site centers. The docking site center was anchored using the residues Lys84, Asn36, and Asp862, located at the bottom of the binding cavity (Figure 2).

After completing the docking simulations, the results were analyzed to establish correlations between each molecule's score and its documented inhibitory activity. The Lys84 residue demonstrated the best reproduction of molecular interactions in the binding site and showed the best correlation with experimental inhibitory activities. A similar procedure was followed for the four scoring functions. The primary goal of this procedure was to enhance the protocol's ability to distinguish highly active compounds and assign a more accurate score. No previous protocol was available for reference. Although the docking scores were not particularly high, the molecules were well-positioned within the pocket defined by the Lys84 residue. The docking cavity is slightly open, as mentioned earlier, which may influence the score attributed by GOLD.

Considering all the molecular docking results for the 64 ChEMBL molecules, the optimal and most reproducible parameters identified involved the use of GOLD v5.07 software with the ChemPLP scoring function and 500 GA runs. Docking was centered on the Lys84 residue in the XPG protein, utilizing the 6TUR crystallographic structure. This combination resulted in the best placement of molecules within the pocket and the highest correlation between docking scores and experimental inhibitory activity across all tested systems.

### Virtual screening campaign

Following the establishment of the docking protocol, the virtual screening campaign (Figure 3) was initiated using the ChemBridge library. The strategic selection of ChemBridge aimed to maximize the chances of identifying novel and potent XPG inhibitors (hits) by comprehensively exploring a chemical space that encompassed a variety of functional groups, scaffolds, and stereochemical configurations.

According to the optimal docking protocol identified earlier in this study, docking-based virtual screening calculations were conducted using GOLD software with the ChemPLP fitness scoring function, 500 GA runs, and Lys84 as the docking site center within a 15 Å radius.

The docking results were filtered based on several criteria: docking score, molecular weight, ligand efficiency, logP, and PAINS scaffolds (Figure 3). Visual assessment was also crucial for selecting hits to ensure proper fitting within the binding site region. These filter cut-offs and ranges were defined based on the evaluation of XPG inhibitors retrieved from the ChEMBL database (Figure 1) and established drug-like rules.

The analysis revealed that for active compounds, a cut-off score of 75 was appropriate, with molecular weights ranging between 200 and 650 Da, and logP less than 5. The restrictions of molecular weight and lopP were expanded beyond the initial dataset to evaluate the possibility of finding compounds with more diverse scaffolds. The compounds previously reported were from the same dataset, so it can be more limitative in terms of synthetized molecules. By enlarging the limits of these two properties while maintaining appropriate drug like values, more molecules are allowed to be analyzed through virtual screening.

After applying the filters described in Figure 3, a visual inspection of 316 compounds was performed, reducing the identification to 61 hit compounds. Visual assessment was crucial to ensure proper fitting within the region. The docking site was not the typical defined cavity with only one point of access, so reducing the hits dataset to a manageable number to visual inspection was essential. The 316 molecules were then subsequently clustered based on structural similarities to ensure maximum scaffold diversity in the final dataset.

The clustering process applied revealed 48 distinct clusters. Clusters 1 and 2, which featured the most prevalent scaffolds, were the most populated, each containing 17 and 13 structures, respectively (see Figure 4). This analysis highlighted the presence of different substituents on a shared scaffold within each cluster. The high number of clusters, each with a relatively small number of molecules, underscores the molecular diversity among potential XPG inhibitors. Molecules from each cluster underwent visual inspection in relation to their pocket placement, guiding compound selection accordingly.

By examining the ranked list of the 316 molecules by their properties, it was observed that despite the high number of clusters, the top-classified molecules did not share the same clusters. A significant number of molecular clusters with only 2 molecules (18 out of 48) were identified, explaining the dispersion of scaffolds between these groups. The evaluation, which included visual inspection and clustering of structurally similar scaffolds using the Murcko Scaffold decomposition method in RDKit, assessed similarity and refined the selection of molecules for this challenging target.

One of the primary goals of this drug design protocol was to identify compounds with a more diverse and broader chemical profile than those previously reported. To assess this diversity, the chemical space of the final dataset of the selected compounds was mapped, as illustrated in Figure 5. This plot depicts the chemical space of compounds from ChEMBL with activities greater than or less than 30 nM, alongside those selected from ChemBridge dataset. Interestingly, there is a discernible dispersion of compounds when comparing those selected from the virtual screening to those from ChEMBL. Compounds from ChEMBL cluster more closely together in two major groups, predominantly because their structures are described by only one group of researchers. The virtual screening campaign described here employed structures without any specific structural constraints tied to other active compounds. The scaffolds of the molecules only needed to adhere to the Lipinski rule profile, allowing the possibility to have a broader and more diverse set of compounds.

In Figure 6, the potential inhibitors selected from the ChemBridge database are compared in terms of their position within the binding site relative to the Lys84 residue of the XPG pocket. The compounds either participated in molecular interactions with the Lys84 residue or were positioned in close proximity to it. For example, CB41 interacts with Asn36, and its exposure to the pocket revealed the importance to the placement of the small molecules. All three examples demonstrated access to the cavity in different directions while keeping the access to the internal region of the pocket. The aromatic rings of the compounds were positioned on the outer side of the pocket, while the tertiary alkyl amine group was oriented towards the inner region of the pocket, with different substituents. It is noteworthy that the Lys84 residue also displays an alkyl amine group side chain in this same region.

### Validation of the putative XPG protein inhibitors in cell-based assays

### Characterization of ERCC5 expression levels in NSCLC cell lines

To select the best NSCLC cellular model for the validation of XPG putative inhibitors, mRNA levels of the *ERCC5* gene across all cell lines were assessed using qRT-PCR. The *ERCC5* gene codes for the XPG protein. A total of six NSCLC cell lines were evaluated. The results pointed the H1299 cell line has the highest *ERCC5* levels, followed by the H1993, HCC827, and H460 cell lines. H1975 and A549 cells have the lowest *ERCC5* levels, respectively (Figure 7). Based on these results, the H1299 cell line was selected for further cell-based assays. This selection was relied on the basis that cell lines expressing higher levels of *ERCC5* would better mimic a clinical situation where tumors are overexpressing this NER component.

### First screening approach to assess the effect of the XPG inhibitors in a NSCLC cell line

After the selection of the most suitable cellular model and acquisition of the proposed XPG inhibitors, a first screening approach was performed by assessing if the inhibitors could enhance cisplatin-induced cytotoxicity. A similar methodological approach was conducted as described in Manguinhas et al. (NPL6). An MTS reduction assay was also selected to measure cell viability in the H1299 cell line after exposure to 1 µM of Cisplatin and 50 µM of each putative inhibitor for 72h (25 µM for CB16-G and CB46-G inhibitors due to solubility issues). Figure 8 presents the results for all putative inhibitors selected from the ChemBridge database.

The concentration of 10 µM of cisplatin was applied to induce a moderate decrease in cell viability, approximately 10-15%, as observed by Manguinhas et al. in H1299 cells. While not causing significant cytotoxicity on its own, this concentration allowed to clearly observe the enhanced cytotoxic effects when combined with the putative inhibitor.

Regarding the putative inhibitors, a concentration of 50 µM was selected, as it represents a balanced compromise between two important considerations: efficacy as NER inhibitors and inherent compound toxicity. Opting for higher concentrations, such as 100 µM, could result in increased cytotoxicity due to the compounds' intrinsic cytotoxic properties. Conversely, reducing the concentration during the initial screening phase might lead to overlooking potentially effective compounds that only demonstrate activity at higher concentrations.

To select the most promising XPG inhibitors, a decision-making process was established similar to Manguinhas et al.. Briefly, the first step of selection was based on the effect of the combination of cisplatin with the putative inhibitor against cisplatin alone. If the cytotoxicity of the combined condition relative to cisplatin alone was lower than 85%, indicating an increase in cisplatin cytotoxicity of at least 15%, the compounds were selected to the next stage. Consequently, 21 compounds were removed from further consideration.

The next step of exclusion was based on each compound intrinsic cytotoxicity. Those causing over 70% loss in cell viability at 50 µM (25 µM for CB 16-G and CB46-G) were removed from consideration. However, they were not discarded at this phase but rather subjected to further investigation at a lower concentration (10 µM - see Figure 9). This step resulted in the exclusion of 15 compounds from the pool of top candidates. From the remaining 25 compounds, the last feature for selection was based on the direct effect of the inhibitor on the combinatory effect, independent of its own cytotoxicity in the cells. Hence, the viability of the inhibitor alone was subtracted from the combinatory condition. Compounds with an effect greater than 10% were considered among the best inhibitors. Consequently, 7 compounds emerged as the best candidates for further assessment: CB9-G, CB20-G, CB22-G, CB23-G, CB41-G, CB46-G, and CB49-G. The process for selection is depicted in Figure 10.

From the screening at 10 µM of the extremely cytotoxic 15 compounds (Figure 9), only CB60-G emerged as a promising compound to boost cisplatin cytotoxicity. Based on this assessment, CB60-G was evaluated with the same criteria proposed in Figure 10, and later selected among the pool of best inhibitors, totaling 8 best inhibitors for further evaluation.

### Validation of the identified XPG inhibitors and evaluation in non-tumoral cell lines

The 8 compounds selected as best inhibitors were further assessed using the cell viability MTS assay. The same experimental conditions were applied as in the previous first screening approach and cisplatin was tested at 1 µM alone and in combination.

The compounds CB9-G, CB20-20, CB22-G, CB23-G, CB41-G, and CB49-G were incubated at 50 µM, CB46-G at 25 µM, and CB60-G at 10 µM. The main goal was to compare the effect of each inhibitor in cisplatin-induced cytotoxicity (Figure 11). Overall, CB9-G, CB20-G, B22-G, CB23-G, CB41-G and CB60-G demonstrated to have no cytotoxicity at the concentrations tested (not statistically significant when compared to the control).

Only CB46-G and CB49-B exhibited a cytotoxicity of around 30% to H1299 cells (p < 0.05). In terms of cisplatin effect alone, loss in cell viability was around 10-15%. When taking into consideration the compounds that indeed had a significant effect in cisplatin-induced cytotoxicity (loss of more than 15% in cell viability), only CB46-G, CB49-G and CB60-G demonstrated to have a sensitizing effect. In fact, CB46-G was able to enhance cisplatin-induced cytotoxicity in 31%, CB49-G in 41%, and CB60-G in 12%.

As a pre-clinical safety assessment, the 8 best inhibitors were tested in a non-tumoral human bronchial epithelial cell line (BEAS-2B). Two complementary cell viability approaches were considered, the MTS reduction and the CV staining assays. Each compound was incubated with the cells for 72h at the same concentrations used in the precious assays.

Based on these results, both cell viability endpoints were in accordance for all the tested inhibitors (Figure 12). Overall, only CB9-G and CB46-G induced a loss in cell viability of more than 50%. For this reason, CB49-G and CB60-G demonstrated to be promising candidates to be used as cisplatin enhancers, with no cytotoxicity for non-tumoral cells.

### Interaction Analysis of Inhibitors with XPG Protein

From the group of 61 tested compounds, 8 were identified as the top inhibitors, prompting a detailed structural analysis to elucidate the potential interaction mechanisms between these molecules and the XPG protein.

Using Murcko Scaffold decomposition in RDKit, the clustering of structurally similar scaffolds was conducted. Only one cluster with more than one molecule was identified, reenforcing the structural variability between scaffolds. In CB23, the hydrogen atom is substituted by a methyl group in CB20, while the rest of the scaffold remains unchanged (Figure 13).

Key molecular interactions between these compounds and the protein involved residues Gly2, Asn36, Gln37, and Arg91, with predominant interactions being hydrogen bonds, hydrophobic interactions, and a π-cation interaction with Arg91. Additionally, CB20 exhibited a hydrophobic interaction with Gln4 and Leu88, which were not identified in CB23 (Figure 13).

Despite their structural similarities, these molecules interacted with various residues (Figure 16A). Analyzing the protein residues involved in the interactions with the small molecules helped to identify key residues that potentially promote XPG inhibition when interacting with the dataset of the eight active molecules (Figure 16).

Predominantly, hydrogen bonds and hydrophobic interactions were observed between these eight compounds and the XPG residues, as depicted in Figure 14B, with only occasional π-cation interactions and salt bridges. Residues Gly2 and Asn36 interacted with the highest number of compounds, suggesting their important role in potentially promoting XPG inhibition. These residues predominantly engage in hydrogen bonding interactions.

To gain a better understanding of the inhibition correlation between the active and inactive compounds from ChEMBL and the inhibitors proposed in this investigation, key molecular interactions with the XPG protein were evaluated (Figures 14 and 15).

Among the molecules with inhibitory activity greater that 30 µM (14 out of 64), classified as inactive, interactions primarily involved Asn36, Glu789, Glu791, Ile33, and Lys84. Although no distinct interaction pattern signature was identified, Glu 789 and Glu791 interactions were notably prominent among inactive compounds. The type of interactions observed were consistent with the pattern described earlier, as illustrated in Figure 16.

For the molecules with inhibitory activity up to 30 µM, classified as active from ChEMBL dataset, residues such as Asn36, Ile33 and Lys84 interacted with the highest number of compounds, suggesting their crucial role in potentially promoting XPG inhibition.

These residues were also identified in the dataset, with Asn36 being particularly prominent. A significant occurrence of hydrogen bonds and hydrophobic interactions were observed. Lys84 was involved in a major group of interactions and had previously been identified as contributing to better performance in the docking study.

### Statistical analysis

The results are presented as Average ± SD and were analyzed with one-way ANOVA multiple comparisons with GraphPad Prism^{®} 9.0. p < 0.05 was considered statistically significant (represented as: *p < 0.05, **p < 0.01, ***p < 0.001, and ****p < 0.0001).

The above-described subject matter is provided as an illustration of this invention and should not be interpreted in a limiting sense. The terminology used to describe preferred embodiments of this invention should not be construed to limit the invention to those particular embodiments.

As used in the description, definite and indefinite articles, in their singular form, are intended to encompass plural forms unless the context of the description explicitly indicates otherwise.

The indefinite articles "a" or "an" should generally be interpreted as "one or more" unless the sense of a singular embodiment is clearly defined in a specific situation.

It will be understood that the terms "comprise" and "include", when used in this description, specify the presence of stated features, elements, components, steps, and operations but do not preclude the presence or addition of one or more other features, elements, components, steps, and operations.

As used throughout this patent application, the term "or" is used in an inclusive sense rather than an exclusive sense unless the exclusive sense is clearly defined in a specific situation. In this context, a phrase such as "X utilizes A or B" should be interpreted as including all relevant inclusive combinations.

As understood in the art, the term "optionally substituted" indicates that the specified group is either unsubstituted or substituted by one or more suitable substituents. A "substituent" that is "substituted" is an atom or group which takes the place of a hydrogen atom on the parent chain or cycle of an organic molecule.

"Cyclyl" as used herein refers to a cyclic structure within a chemical compound. This term denotes a monovalent radical or substituent derived from a cyclic compound, where one hydrogen atom has been removed to create a point of attachment for further chemical bonding. It includes both carbocyclic (entirely carbon-based rings) and heterocyclic (rings containing non-carbon atoms, such as nitrogen, oxygen, or sulfur) structures.

"Aryl" as used herein refers to a ring system having one or more aromatic rings. Representative examples of aryl include azulenyl, indanyl, indenyl, naphthyl, phenyl, tetrahydronaphthyl, mesityl, thienyl, and the like.

"Heteroaryl" means a cyclic, aromatic hydrocarbon in which one or more carbon atoms have been replaced with heteroatoms (e.g., N, O or S). If the heteroaryl group contains more than one heteroatom, the heteroatoms may be the same or different.

"Hydrogen bond donor" as used herein means a group that under suitable conditions can ionize to release a H+ ion and to produce a negatively charged species. Examples of groups of this type include inorganic acids, sulfonic acids, carboxylic acids, anionic amino acids, hydroxyl acids, fatty acids for insoluble salts.

"Hydrogen bod acceptor" as used herein means a group that under suitable conditions can react with H+ ion to form a positively charged species. Examples of groups of this type include organic amines, cationic amines and bases for insoluble salts.

"Alkoxy" as used herein, refers to an alkyl group, as defined herein, appended to the parent molecular moiety through an oxy group, as defined herein.

An "amine" or "amino" is intended to mean the group -NH2. "Primary amines" have one of three hydrogen atoms replaced by an alkyl or aromatic group. "Secondary amines" have two organic substituents bound to the nitrogen together with one hydrogen.

An "amide" as used herein refers to a functional group having a carbonyl group (C=O) linked to a nitrogen atom (N), or an organic compound that contains this group.

The term "oxo" as used herein, refers to a =O moiety. The term "oxy" as used herein, refers to a -O- moiety.

"Nitro" refers to the organic compound functional group -NO2.

"Carbonyl" is a functional group having a carbon atom double bonded to an oxygen atom (-C=O).

"Carboxy" as used herein refers to a -COOH functional group, also written as - CO2H or -(C=O)-OH.

All changes, as long as they do not modify the essential characteristics of the following claims, should be considered within the scope of protection of this invention.

### Citation List

The citation list is as follows:

### Patent Literature

PTL1: US20210093730A1
PTL2: US20240207300A1
PTL3: US2020237763A1
PTL4: US11584755B2

### Non-Patent Literature

NPL1: Burgess JT, Rose M, Boucher D, Plowman J, Molloy C, Fisher M, O'Leary C, Richard DJ, O'Byrne KJ, Bolderson E. (2020). The Therapeutic Potential of DNA Damage Repair Pathways and Genomic Stability in Lung Cancer. Front Oncol. Jul 28;10:1256. doi: 10.3389/fonc.2020.01256. PMID: 32850380; PMCID: PMC7399071.
NPL2: Burgess JT, Croft LV, Wallace NC, Stephenson SA, Adams MN, Ashton NW, Richard DJ. (2014). DNA Repair Pathways and their Therapeutic Potential in Lung Cancer. Lung Cancer Management, 3(2), 159-173. https://doi.org/10.2217/lmt.14.12
NPL3: Barros, A.C.D.; Takeda, A.A.S.; Dreyer, T.R.; Velazquez-Campoy, A.; Kobe, B.; Fontes, M.R.M. Structural and Calorimetric Studies Demonstrate that Xeroderma Pigmentosum Type G (XPG) Can Be Imported to the Nucleus by a Classical Nuclear Import Pathway via a Monopartite NLS Sequence. J. Mol. Biol. 2016, 428, 2120-2131.
NPL4: Tsutakawa, S.E.; Sarker, A.H.; Ng, C.; Arvai, A.S.; Shin, D.S.; Shih, B.; Jiang, S.; Thwin, A.C.; Tsai, M.S.; Willcox, A.; et al. Human XPG nuclease structure, assembly, and activities with insights for neurodegeneration and cancer from pathogenic mutations. Proc. Natl. Acad. Sci. U. S. A. 2020, 117, 14127-14138.
NPL5: González-Corrochano, R.; Ruiz, F.M.; Taylor, N.M.I.; Huecas, S.; Drakulic, S.; Spinola-Amilibia, M.; Fernández-Tornero, C. The crystal structure of human XPG, the xeroderma pigmentosum group G endonuclease, provides insight into nucleotide excision DNA repair. Nucleic Acids Res. 2020, 48, 9943-9958.
NPL6: Manguinhas, R.; Serra, P.A.; Soares, R.B.; Rosell, R.; Gil, N.; Oliveira, N.G.; Guedes, R.C. Unveiling Novel ERCC1-XPF Complex Inhibitors: Bridging the Gap from In Silico Exploration to Experimental Design. Int. J. Mol. Sci. 2024, 25.

## Claims

1. XPG inhibitor compounds comprising:
at least one optionally substituted aromatic or heteroaromatic ring;
none or 1 hydrogen bond donors selected from at least one of a hydroxyl group, a ketone group, a thiol group and a nitrogen-containing group;
at least 4 hydrogen bond acceptors selected from at least one of oxygen, nitrogen and sulfur;
wherein the molecular weight ranges between 200 and 300 g/mol;
and / or a pharmaceutically acceptable salt, solvate or tautomer thereof for use as a medicament in the treatment of cancer, tumors and / or metastases in combination with at least one platinum-based anticancer agent, wherein the compound and the Lys84 residue of the XPG protein participate in a molecular interaction to inhibit XPG activity.

2. XPG inhibitor compounds comprising:
at least one optionally substituted phenyl, furanyl, pyrrolyl, thienyl, thiophenyl, oxazolyl, indazolyl, isoxazolyl, thiazolyl, isothiazolyl, imidazolyl, pyrazolyl, triazolyl, pyridyl, pyridinyl, pyrazolyl, dihydrothiadiazolyl, dioxazolyl, oxadiazolyl, pyrazinyl, pyrimidinyl, pyridazinyl, piperazinyl, triazinyl, indolyl, quinolinyl, isoquinolinyl, purpyrrolyl, benzimidazolyl, benzoxazolyl, benzothiazolyl, pteridinyl, azepanyl, diazinanyl, pyrrolidinyl, imidazolyl, imidazolidinyl, morpholinyl or other mono- or poly aromatic or non-aromatic homocyclic or heterocyclic compound;
none or 1 hydrogen bond donors selected from at least one of a hydroxyl group, a ketone group, a thiol group and a nitrogen-containing group selected from substituted or unsubstituted amino group selected from primary or secondary amine or amide;
at least 4 hydrogen bond acceptors selected from at least one of oxygen, nitrogen and sulfur, the oxygen being found in carbonyl groups, hydroxyl groups, ether groups or esters groups, the nitrogen being found in amines, amides and heteroaryl groups, and the sulfur being found in sulfonyl and thiocarbonyl groups;
wherein the molecular weight ranges between 200 and 300 g/mol;
and / or a pharmaceutically acceptable salt, solvate or tautomer thereof for use as a medicament in the treatment of cancer, tumors and / or metastases in combination with at least one platinum-based anticancer agent, wherein the compound and the Lys84 residue of the XPG protein participate in a molecular interaction to inhibit XPG activity.

3. XPG inhibitor compounds selected from:
2-(2-{[4-(acetylamino)phenyl]amino}-2-oxoethoxy)-N-[2-(2-isopropyl-5-methylphenoxy)ethyl]benzamide 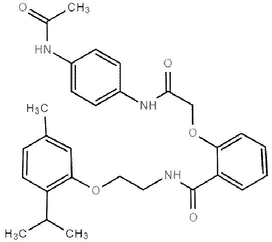
N-[3-(1H-imidazol-1-yl)propyl]-3-{[(5-methyl-2-thienyl)methyl]amino}-5-(1-pyrrolidinylsulfonyl)benzamide 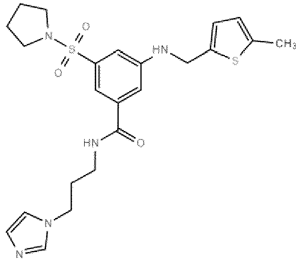
1-[3-(2-{4-[(3,5-dimethyl-1H-pyrazol-1-yl)acetyl]-l-piperazinyl}ethoxy)phenyl]-N-methyl-N-(2-thienylmethyl)methanamine 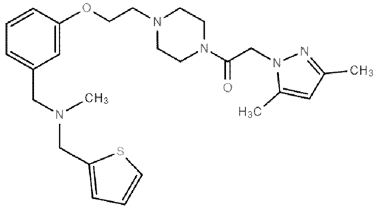
N-[3-(1H-imidazol-1-yl)propyl]-3-(1-pyrrolidinylsulfonyl)-5-[(3-thienylmethyl)amino]benzamide 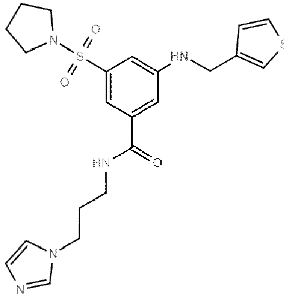
N-({8-[4-(3-hydroxy-3-methyl-1-butyn-1-yl)benzyl]-1-oxa-8-azaspiro[4.5]dec-2-yl}methyl)-1,3-dimethyl-1H-pyrazole-5-carboxamide 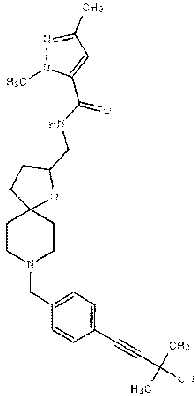
1,1'-[1,4-phenylenebis(oxy-4,1-phenylene)]di(2,5-pyrrolidinedione) 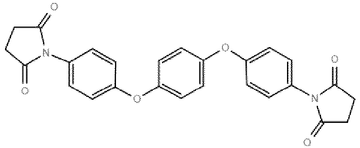
Methyl 1-benzyl-5-[(cyclohexylmethyl)amino]-3-[(3-pyridinylcarbonyl) amino]-1H-pyrrolo[2,3-b]pyridine-2-carboxylate 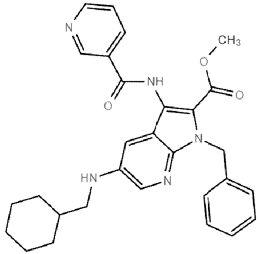
N-[(2R*,3R*)-2-(benzyloxy)-1'-(1H-indol-6-ylcarbonyl)-2,3-dihydrospiro[indene-1,4'-piperidin]-3-yl] acetamide 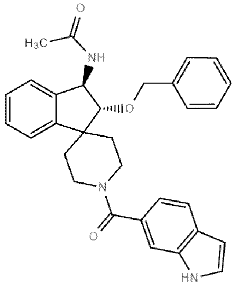
and / or a pharmaceutically acceptable salt, solvate or tautomer thereof for use as a medicament in the treatment of cancer, tumors and / or metastases in combination with platinum-based anticancer agent, wherein the compound and the Lys84 residue of the XPG protein participate in a molecular interaction to inhibit XPG activity.

4. XPG inhibitors compounds and / or pharmaceutically acceptable salt, solvate or tautomer thereof for use in the treatment of cancer, tumours and / or metastases according to any of claims 1-3 wherein the platinum-based anticancer agent is selected from the group consisting of cisplatin, carboplatin, oxaliplatin, nedaplatin, lobaplatin, satraplatin, and heptaplatin.

5. XPG inhibitors compounds and / or pharmaceutically acceptable salt, solvate or tautomer thereof for use according to any of claims 1-4 wherein the cancer is non-small cell lung cancer.

6. Pharmaceutical composition comprising an effective amount of at least one XPG inhibitor compound according to any of claims 1-3 and / or a pharmaceutically acceptable salt, solvate or tautomer thereof and excipients.

7. Pharmaceutical composition, according to claim 6, further comprising at least one platinum-based anticancer agent.

8. Pharmaceutical composition, according to claim 7, wherein the platinum-based anticancer agent is selected from the group consisting of cisplatin, carboplatin, oxaliplatin, nedaplatin, lobaplatin, satraplatin, and heptaplatin.

9. Kit comprising separate packs of
(a) an effective amount of at least one XPG inhibitor compound according to any of claims 1-3 and / or a pharmaceutically acceptable salt, solvate or tautomer thereof, and
(b) an effective amount of at least one platinum-based anticancer agent, wherein the platinum-based anticancer agent is selected from the group consisting of cisplatin, carboplatin, oxaliplatin, nedaplatin, lobaplatin, satraplatin, and heptaplatin.
